# EUROPEAN PATENT SPECIFICATION

(11) **EP 3 973 947 B1**
(45) Date of publication and mention of the grant of the patent: **13.08.2025**
(21) Application number: 21198777.1
(22) Date of filing: 24.09.2021
(51) Int. Cl.: A61K 8/36, A61K 8/39, A61Q 5/04

(54) **COMPOSITION, PROCESS AND KIT FOR SEMI-PERMANENT STRAIGHTENING KERATIN FIBERS**
ZUSAMMENSETZUNG, VERFAHREN UND KIT ZUM SEMIPERMANENTEN STRECKEN VON KERATINFASERN
COMPOSITION, PROCÉDÉ ET KIT DE LISSAGE SEMI-PERMANENT DE FIBRES KÉRATINIQUES

(30) Priority: 25.09.2020 EP 20198409
(43) Date of publication of application: 30.03.2022
(73) Proprietor: Kao Germany GmbH, 64297 Darmstadt (DE)
(72) Inventor: Baar, Moritz, Cincinnati, 45214-1729 (US)

(56) References cited:
- EP-A1- 3 659 577
- WO-A1-2014/131470
- DATABASE GNPD [online] MINTEL; 3 October 2018 (2018-10-03), ANONYMOUS: "Brilliance Creator", XP055785486, retrieved from https://www.gnpd.com/sinatra/recordpage/5916957/ Database accession no. 5916957

## Description

The present invention relates to a composition, process and kit for semi-permanent straightening keratin fibers especially human hair, which involves using an aqueous composition comprising a carboxylic acid and a nonionic surfactant.

Changing ones hair shape has always been in vogue. Already in the ancient Egyptian era, long, straight hair was popular. Women, which were not blessed with this kind of natural hair already used in fire heated iron plates to get their hair in the desired shape. This procedure often led to severe burns to the face and hands. It was not before the early 19th century that straightening irons became more professional with the invention of a hair tong made by Marcel Grateau. Nowadays, hot tools like straightening irons as well as blow dryers are more popular than ever before. They got into the focus of consumers in both salon and mass market. These tools lead to satisfying results as they work at high temperatures (up to 230°C for a straightening iron). When dry hair is getting in contact with the hot iron and a proper pressure is applied at the same time, the hair shape can be fixed for a certain amount of time until hair is humidified again. Unfortunately, application of such hot temperature leads to extreme hair damage and thus results in a bad hair feel after their usage. Consequently, there is a huge need of products, which allow straightening of hair more effectively to limit the contact time between iron and hair while reducing damage and leaving a pleasant hair feel after application at the same time. Additionally, there is a need for completely abandoning straight irons from the daily styling routine in order to achieve efficiently a smooth and even result with reduced volume by only blow-drying the hair. Glyoxylic acid is a known ingredient for hair cosmetics, which allows long-lasting curl relaxation and volume reduction especially in combination with straightening irons at high temperatures. Combined with only blow-drying of the hair, the volume reduction and especially curl relaxation is way less effective with lower durability and often higher amounts of Glyoxylic Acid are needed. For both applications, there is a general need to reduce the usage amount of Glyoxylic Acid to avoid unwanted side effects. This includes a sensitizing potential as well as a stiff hair feel and an unpleasant smell that comes with the application of higher amounts of Glyoxylic Acid. To achieve the maximum results with the use of as little Glyoxylic Acid as possible it is of advantage not to rinse out the product before blow-drying and optimal straightening the hair. Unfortunately, a composition containing Glyoxylic Acid leaves sticky residues on the hair during drying and makes handling hair often very difficult; especially combing hair becomes relatively difficult.

In this respect, WO 2011/104282 describes a process for semi-permanent hair straightening, which involves applying a composition comprising an α-keto acid onto the hair, leaving the composition in contact with the hair for 15 to 120 minutes, drying the hair and straightening the hair with a straightening iron at a temperature of 200 +-50°C.

Furthermore, WO 2012/010351 describes a treatment for semi-permanent straightening of curly, frizzy or wavy hair by applying a solution of glyoxylic acid in combination with mechanical straightening, using a straightening iron at a temperature of 200 +- 30°C. After the treatment, the hair is said to retain its shape for at least six consecutive washings

WO 2014/072645A1 is a post-published document according to Article 54(3) EPC, which relates to a hair straightening procedure involving the application of a glyoxylic acid-containing composition onto the hair, followed by the application of a composition comprising a fatty alcohol or a cationic conditioning agent and the use heat, e.g., by means of a straightening iron. The cationic conditioning agent may be a cationic polymer. The glyoxylic acid-containing compositions may also include a nonionic cellulose-based polymer such as methylcellulose or hydroxyethylcellulose.

WO 2014/072479A2 is a post-published document according to Article 54(3) EPC and concerns a hair straightening composition comprising a certain dicarbonyl compound such as glyoxylic acid in combination with at least one cationic and/or at least one amphoteric polymer. The described compositions also may comprise hydroxypropyl methyl cellulose.

The present invention provides a composition that enables efficient straightening while providing an easy handling when blow-drying and / or straight ironing due to a less sticky hair feel. With the use of the composition of the present invention prior to hair straightening as well as blow drying process, semi-permanent hair straightening result and a volume down effect are achieved while still keeping good handling of hair during and as well as after blow drying and/or straightening.

The inventor of the present invention has unexpectedly observed that an aqueous composition comprising an organic carboxylic acid and nonionic surfactant makes possible semi-permanent straightening and volume reduction of hair possible while achieving improved combing of the hair.

The first object of the present invention is an aqueous composition for semi-permanent straightening keratin fibers, especially human hair, comprising a concentration in the range of 0.5 to 30 % by weight of glyoxylic acid ; and a concentration in the range of 0.5 to 15 % by weight of a polyethylene glycol alkyl ether according to the general structure

R₂ - O - (CH₂CH₂O)ₙ H

wherein R₂ is a saturated alkyl chain with 9 to 16 C atoms and n is a value in the range of 8 to 16, wherein it has a pH 4.0 or below, wherein all concentrations are calculated to the total of the composition, wherein the composition is free of sulfur-based reducing agents.

The second object is a process for semi-permanent straightening keratin fibers, especially human hair, comprising the following steps performed in this order:
(a) application of the composition of the present invention onto the keratin fibes, especially human hair, preferably in a weight ratio of hair to composition of 0.5:2 to 2:0.5;
(b) leaving the composition on the hair for 5 to 120 minutes at a temperature in the range of 20 to 50°C;
(c) optionally rinsing off the composition;
(d) drying the hair with a blow dryer;
(e) optionally treating the hair with an iron having a surface temperature of 180 +- 50°C, preferably 170 to 200°C, and
(f) optionally rinsing off and/or shampooing the hair and drying.

The third object is kit for keratin fibers especially human hair comprising the composition of the present invention. examples are glyoxylic acid, pyruvic acid and 2-ketobutyric acid.

The glyoxylic acid may be comprised in the composition in its free acid form. The carbonyl group adjacent to the acid group of the acid may also be present in the hydrate form. Apart from the free acid form and the hydrate thereof, salts of the acid or the hydrate may also be used.

Glyoxylic acid (H-CO-COOH) in aqueous solution is almost quantitatively present as the hydrate (H-C(OH)₂-COOH) Besides, the hydrate may also condense to dimers. A salt of the carboxylic acid may also be used. As examples, alkali metal salts such as the sodium or potassium salt, alkaline earth metal salts such as the magnesium salt or the calcium salt and tertiary and quaternary ammonium salts may be mentioned.

Conventional permanent hair shaping/straightening techniques are based on the reorganization of the disulfide bridges and involve a cleavage of the disulfide bonds either by using a sulfur-based reducing agent or an alkali agent, followed by the shaping of the hair and the formation of new bonds (i.e., disulfide bonds formed by the action of an oxidizing agent or thioether bonds, respectively). In contrast to these permanent straightening methods, the present invention does not utilize cleavage of the disulfide bonds and fixing the bonds in the new shape. Therefore, the semi-permanent straightening composition is free of sulfur based reducing agents. pH of the aqueous composition is 4.0 or less, preferably in the range of 1 to 3.5, more preferably 1 to 3 and more preferably 1.5 to 3, as measured directly and at ambient temperature (25°C). The pH of the composition may be adjusted using known alkaline solutions, preferably with sodium hydroxide solution.

The concentration of polyethylene glycol alkyl ether of the above general structure is in the range of 0.5 to 15% by weight, preferably 0.5 to 7.5% by weight, calculated to the total of the composition.

Suitable non-limiting and preferred polyethylene glycol alkyl ether is C12-13 pareth-9. In a further preferred embodiment of the present invention, the aqueous composition comprises foaming surfactant at a total concentration 10% by weight or less, calculated to the total of the composition.

In a further preferred embodiment of the present invention the aqueous composition of the present invention comprises one or more thickening polymers preferably selected from anionic, cationic, amphoteric and nonionic polymers having a viscosity of at least 500 mPa·s measured at a polymer concentration of 1% by weight, in water and at 20°C with a Brookfield viscometer.

Suitable non-limiting non-ionic polymers are include neutral polysaccharides and derivatives such as ethers or esters thereof. In this respect, neutral gums such as, sclerotium gum, guar gum, hydroxypropyl guar, cellulose ethers such as hydroxyethylcellulose (HEC), methyl hydroxyethylcellulose (MHEC), ethyl hydroxyethylcellulose (EHEC), methyl ethyl hydroxyethylcellulose (MEHEC), hydroxypropylcellulose (HPC), hydroxypropylmethylcellulose (HPMC), hydrophobically modified derivatives thereof such as HM-EHEC, starch and dextrins may be mentioned.

Non-limiting examples for the anionic polymer include anionic polysaccharides and derivatives thereof, such as alginate, pectin, hyaluronate, anionic gums such as xanthan gum, dehydroxanthan gum, hydroxypropyl xanthan gum, gum arabic, gum karaya or gum tragacanth, or anionic cellulose derivatives such as carboxymethyl cellulose (CMC). Further examples include synthetic anionic polymers such as polyacrylic acid or copolymers containing acrylic acid in combination with neutral vinylic and/or acrylic monomers, and salts thereof such as sodium polyacrylate.

Non-limiting examples of cationic polymers include cationized cellulose, cationic starch, cationic guar gum, a vinylic or (meth)acrylic polymer or copolymer having quaternary ammonium side chains, (meth)acrylate/aminoacrylate copolymer, amine substituted polyacrylate crosspolymers, a polymer or copolymer of a diallyl quaternary ammonium salt, and quaternized polyvinylpyrrolidone. As an example of the vinylic or (meth)acrylic polymer or copolymer having quaternary ammonium side chains, poly(2- methacryloxyethyltrimethylammonium chloride) (Polyquaternium- 37) may be mentioned. Specific examples of the quaternized polyvinylpyrrolidone include quaternary ammonium salts synthesized from a copolymer of vinylpyrrolidone (VP) and dimethylaminoethyl methacrylate, and diethyl sulfate (polyquaternium-11). As an example of the (meth)acrylate/aminoacrylate copolymer, Acrylates/Aminoacrylates/Cl0-30 Alkyl PEG-20 Itaconate Copolymer may be mentioned. As an example of the amine substituted polyacrylate crosspolymers, Polyacrylates-1 Crosspolymer may be mentioned. Specific examples of the cationized cellulose include a polymer of a quaternary ammonium salt obtained by adding glycidyltrimethylammonium chloride to hydroxyethylcellulose (polyquaternium 10), and a hydroxyethylcellulose/ dimethyldiallylammonium chloride copolymer (polyquaternium- 4), and a polymeric quaternary ammonium salt of hydroxyethyl cellulose reacted with a trimethyl ammonium substituted epoxide and a lauryl dimethyl ammonium substituted epoxide (polyqauternium-67).

As examples for the amphoteric polymer, carboxyl-modified or sulfonate-modified cationic polysaccharides such as carboxymethylchitosan may be mentioned. Further examples include copolymers of cationic vinylic or (meth)acrylic monomers with (meth)acrylic acid, such as dimethyldiallylammonium chloride/acrylic acid copolymer (polyquaternium-22).

Anionic poklymers are preferred and in particular the thickening polymer is selected from xanthan gum, hydroxypropyl xanthan gum and dehydro xanthan gum and their mixtures.

The concentration of the one or more thickening polymers is in the range of 0.01 to 5 wt.%, preferably 0.05 to 4 wt.%, more preferable 0.1 to 3 wt.%, and more preferably 0.25 to 2.5 wt.% calculated to the total of the composition.

The aqueous composition comprises one or more quaternary ammonium surfactant of the general formula 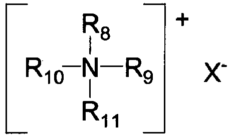 wherein R₈ and R₉ are independent from each other a saturated or unsaturated, branched or straight alkyl chain with 8-22 C atoms or

R₁₂-CO-NH-(CH₂)ₙ

wherein R₁₂ is a saturated or unsaturated, branched or straight alkyl chain with 7-21 C atoms and n is an integer of 1 - 4, or

   R₁₂CO-O-(CH₂)ₙ
wherein R12 and n are same as above,
R10 and R11 are independent from each other an alkyl group with 1 to 4 carbon atoms, hydroxyl alkyl chain with 1 to 4 carbon atoms, or ethoxy or propoxy group with a number of ethoxy or propoxy groups varying in the range of 1 to 4, and X is chloride, bromide, methosulfate or ethosulfate.

The total concentration of one or more ammonium surfactant of the above general formula is in the range of 0.01 to 5%, preferably 0.1 to 3, more preferably 0.1 to 2% by weight, calculated to the total of the composition.

The composition preferably comprises one or more oils, preferably selected from silicone oils, natural oils, mineral oil and synthetic oils.

The total concentration of one or more oils is in the range of 0.01 to 30 wt.%, more preferably 0.05 to 20 wt.%, and even more preferably 0.1% to 10 wt.%, calculated to the total of the composition.

The suitable silicone oils are dimethylpolysiloxane, and modified silicone (for example, amino-modified silicone, fluorine-modified silicone, alcohol-modified silicone, polyether-modified silicone, epoxy-modified silicone, polyoxazoline silicone (as described in JP A 2-276824), or alkyl-modified silicone), but dimethylpolysiloxane, polyether-modified silicone and aminomodified silicone are preferred.

The dimethylpolysiloxane may be any cyclic or non-cyclic dimethylsiloxane polymer, and examples thereof include SH200 series, BY22-019, BY22-020, BYII-026, B22-029, BY22-034, BY22-050A, BY22-055, BY22-060, BY22-083, FZ-4188 (all by Dow Corning Toray Co., Ltd.), KF-9008, KM-900 series, MK-15H, and MK-88 (all by ShinEtsu Chemical Co., Ltd.).

The polyether-modified silicone may be any silicone having a polyoxyalkylene group, and the group constituting the polyoxyalkylene group may be an oxyethylene group or an oxypropylene group. More specific examples include KF-6015, KF-945A, KF-6005, KF-6009, KF-6013, KF-6019, KF-6029, KF- 6017, KF-6043, KF-353A, KF-354A, KF-355A (all by Shin-Etsu Chemical Co., Ltd.), FZ-2404, SS-2805, FZ-2411, FZ-2412, SH3771M, SH3772M, SH3773M, SH3775M, SH3749, SS-280X series, BY22-008 M, BYII-030, and BY25-337 (all by Dow Corning Toray Co., Ltd.) .

The amino-modified silicone may be any silicone having an amino group or an ammonium group, and examples thereof include an amino-modified silicone oil having all or a part of the terminal hydroxyl groups capped with a methyl group or the like, and an amodimethicone which does not have the terminals capped. A preferred example of the amino-modified silicone may be a compound represented by the following formula: 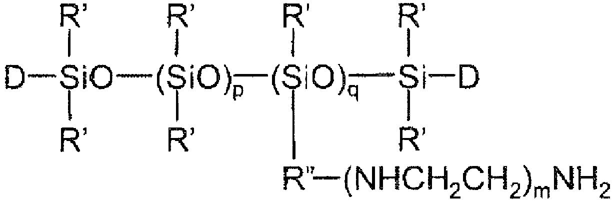 wherein R' represents a hydroxyl group, a hydrogen atom or Rx; Rx represents a substituted or unsubstituted monovalent hydrocarbon group having 1 to 20 carbon atoms; D represents Rx, R"- (NHCH2CH2 )mNH2 , ORx, or a hydroxyl group; R" represents a divalent hydrocarbon group having 1 to 8 carbon atoms; m represents a number from Oto 3; p and q represent numbers, the sum of which is, as a number average, equal to or greater than 10 and less than 20,000, preferably equal to or greater than 20 and less than 3000, more preferably equal to or greater than 30 and less than 1000, and even more preferably equal to or greater than 40 and less than 800.

Specific examples of suitable commercially available products of the amino-modified silicone include amino-modified silicone oils such as SF8452C, SS-3551 (all by Dow Corning Toray Co., Ltd.), KF-8004, KF-867S, and KF-8015 (all by ShinEtsu Chemical Co. , Ltd.); and amodimethicone emulsions such as SM8704C, SM8904, BY22-079, FZ-4671, and FZ-4672 (all by Dow corning Toray Co., Ltd.).

Further oil suitable oil components are hydrocarbon oils having at least 10 carbon atoms, a polyolefin, a fatty acid ester, a fatty acid amide, a polyalkylene glycol, and mixtures thereof. Examples of the hydrocarbon oil include a cyclic hydrocarbon, a linear aliphatic hydrocarbon (saturated or unsaturated), and a branched aliphatic hydrocarbon (saturated or unsaturated), and polymers or mixtures thereof are also included. The linear hydrocarbon oil preferably has 12 to 19 carbon atoms. The branched hydrocarbon oil includes hydrocarbon polymers, and preferably has more than 19 carbon atoms.

The polyolefin is a liquid polyolefin, more preferably a liquid poly-a-olefin, and even more preferably a hydrogenated liquid poly-a-olefin. The polyolefin used herein is prepared by polymerizing an olefin monomer having 4 to 14 carbon atoms, and preferably 6 to 12 carbon atoms.

The fatty acid ester may be, for example, a fatty acid ester having at least 10 carbon atoms. Examples of such a fatty acid ester include esters having a hydrocarbon chain derived from a fatty acid and an alcohol (for example, monoesters, polyhydric alcohol esters, or di- and tricarboxylic acid esters). The hydrocarbon group of these fatty acid esters may have another compatible functional group such as an amide group or an alkoxy group as a substituent, or the hydrocarbon group may be covalently bonded to those functional groups.

More specifically, an alkyl and alkenyl ester of a fatty acid having a fatty acid chain having 10 to 22 carbon atoms, a carboxylic acid ester of an aliphatic alcohol having an aliphatic chain derived from an alkyl and/or alkenyl alcohol having 10 to 22 carbon atoms, and a mixture thereof are suitably used. Specific examples of these preferred fatty acid esters include isopropyl isostearate, hexyl laurate, isohexyl laurate, isohexyl palrnitate, isopropyl palmitate, isopropyl rnyristate, decyl oleate, isodecyl oleate, hexadecyl stearate, decyl stearate, dihexadecyl adipate, lauryl lactate, myristyl lactate, cetyl lactate, oleyl stearate, oleyl oleate, oleyl myristate, lauryl acetate, cetyl propionate and dioleyl adipate.

Further suitable oil components are natural oils such as paraffin oil and natural triglycerides. Suitable natural triglycerides are argan oil, shea butter oil, karite oil, olive oil, almond oil, avocado oil, ricinus oil, coconut oil, palm oil, sesame oil, peanut oil, sunflower oil, peach kernel oil, wheat germ oil, macadamia nut oil, macadamia oil, night primrose oil, jojoba oil, castor oil, soya oil, lanolin, passiflora oil, black cumin oil, borage oils, grapeseed oil, hempseed oil, kukui nut oil, and rosehip oil.

The composition may be in the form of an emulsion.

The composition may further comprise one or more fatty alcohol, preferably at a total concentration in the range of 0.1 to 20 wt.%, more preferably 0.2 to 15 wt.%, most preferably 0.5 to 10 wt.%, calculated to the total of the composition.

The suitable fatty alcohols are having an alkyl chain which may be saturated or unsaturated, straight or branched and with 8 to 22 C atoms. Suitable non-limiting examples are cetyl alcohol, stearyl alcohol, behenyl alcohol, and mixtures thereof.

The semi-permanent straightening process is carried out comprising the following steps performed in this order:
(a) application of the aqueous composition of the present invention onto the hair, preferably in a weight ratio of hair to composition of 0.5:2 to 2:0.5;
(b) leaving the composition on the hair for 5 to 120 minutes at a temperature in the range of 20 to 50°C;
(c) optionally rinsing off the hair;
(d) drying the hair with a blow dryer;
(e) optionally treating the hair with an iron having a surface temperature of 180 +- 50°C, preferably 170 to 200°C, and
(f) optionally rinsing off and/or shampooing the hair and drying.

In step (a) of the process of the present invention, the aqueous composition composition is applied to the hair. The application weight ratio of hair to composition is 0.5:2 to 2:0.5, preferably 0.5:1 to 1:0.5, more preferably about 1:1. Subsequent to the application, the straightening composition is left on the hair for 5 to 120 minutes, preferably 5 to 90 minutes, more preferably 10 to 60 minutes and more preferably 15 to 45 minutes at a temperature range of 20 to 50°C, preferably at ambient temperature (step (b)). Then, the composition is optionally rinsed off from hair (step (c)). In subsequent step (d), the hair is dried with a blow drier. It is preferable to dry the hair under continuous combing in order to prevent entanglement of the hair. Subsequent to the drying, the hair may be treated with an iron having a surface temperature of 180±50°C, preferably 170 to 200°C. A usual straightening iron may be used for this purpose (step (e)). Furthermore, the hair may optionally be rinsed off with water and/or shampooed and dried again (step ( f) ).

The aqueous composition is provided to the user in a kit, which comprises the composition of the present invention and optionally one or more of additional compositions and /or hair appliances such as a hair drier. In the preferred form, the kit comprises the composition of the present invention and a hair drier and optionally a hair straightening iron.

### Examples

### Example 1

| | Concentration % by weight | | | | |
|---|---|---|---|---|---|
| | A | B | C | D | E |
| Water | | | q.s. to 100 | | |
| Dehydroxanthan gum | 0,6 | 0,6 | 0,6 | 0,6 | 0,6 |
| Propylene glycol | 1 | 1 | 1 | 1 | 1 |
| Glyoxylic acid | 10 | 10 | 10 | 10 | 10 |
| Sodium hydroxide | 1 | 1 | 1 | 1 | 1 |
| Fragrance | 0,5 | 0,5 | 0,5 | 0,5 | 0,5 |
| Cetyl PEG/PPG 10/1 dimethicone | - | 1,5 | - | - | - |
| Butylene glycol | - | - | 1,5 | - | - |
| PPG-3 Caprylyl ether | - | - | - | 1,5 | - |
| C12-13-Pareth-9 | - | - | - | - | 1,5 |

The pre-damaged hair streaks weight 2 g by means of permanent shaping and bleaching were semi-permanently straightened with the above compositions. Therefore, the hair streaks were washed with a commercially available shampoo composition and dried. Subsequently, above compositions ere applied to different streaks at a weight ratio of composition to hair streak 2 to 1 and left at 40°C for 15 min and dried with a blow drier. It was observed that the streak treated with the composition E was well straightened and combable. The other streaks looked straightened as well but all of them (four streaks) were difficult to comb. This shows clearly that the composition of the present invention does provide hair improved combability

## Claims

1. An aqueous composition for semi-permanent straightening and/or volume reduction of keratin fibers, especially human hair, **characterized in that** it comprises glyoxylic acid, hydrates thereof, or their salts at a concentration in the range of 0.5 to 30% by weight,
and a polyethylene glycol alkyl ether according to the general structure
R₂ - O - (CH₂CH₂O)ₙ H
wherein R₂ is a saturated alkyl chain with 9 to 16 C atoms and n is a value in the range of 8 to 16 at a concentration in the range of 0.5 to 15% by weight, and
it has a pH 4.0 or below,
wherein all concentrations are calculated to the total of the composition,
wherein the composition is free of sulfur-based reducing agents.

2. The composition according to claim 1 **characterized in that** the pH of the composition is 3 or less, especially in the range of 1 to 3.

3. The composition according to any of the preceding claims **characterized in that** polyethylene glycol alkyl ether has an alkyl chain length of 11 to 15 C atoms, preferably 12 to 13 C atoms and n is a value of 8 to 14 and especially 9 to 12.

4. The composition according to any of the preceding claims **characterized in that** polyethylene glycol alkyl ether is C12-13 pareth-9.

5. The composition according to any of the preceding claims **characterized in that** it comprises foaming surfactant at a total concentration 10% by weight or less, calculated to the total of the composition.

6. The composition according to any of the preceding claims **characterized in that** it comprises polyethylene glycol alkyl ether at a concentration in the range of 0.5 to 7.5% by weight, calculated to the total of the composition.

7. The composition according to any of the preceding claims **characterized in that** it comprises one or more thickening polymers, preferably selected from anionic polymers having a viscosity of at least 500 mPa·s measured at a polymer concentration of 1% by weight, in water and at 20°C with a Brookfield viscometer, in particular selected from xanthan gum, hydroxypropyl xanthan gum and dehydro xanthan gum.

8. The composition according to any of the preceding claims **characterized in that** it comprises quaternary ammonium surfactant of the general formula 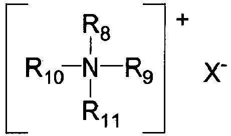
wherein R₈ and R₉ are independent from each other a saturated or unsaturated, branched or straight alkyl chain with 8-22 C atoms or
R₁₂-CO-NH-(CH₂)ₙ
wherein R₁₂ is a saturated or unsaturated, branched or straight alkyl chain with 7-21 C atoms and n is an integer of 1 - 4, or
R₁₂CO-O-(CH₂)ₙ
wherein R₁₂ is a saturated or unsaturated, branched or straight alkyl chain with 7-21 C atoms and n is an integer of 1 - 4,
R₁₀ and R₁₁ are independent from each other an alkyl group with 1 to 4 carbon atoms, hydroxyl alkyl chain with 1 to 4 carbon atoms, or ethoxy or propoxy group with a number of ethoxy or propoxy groups varying in the range of 1 to 4, and X is chloride, bromide, methosulfate or ethosulfate.

9. The composition according to any of the preceding claims **characterized in that** it comprises one or more oils, preferably selected from silicone oils, natural oils, mineral oil and synthetic oils.

10. The composition according to any of the preceding claims, **characterized in that** it is an emulsion.

11. The composition according to any of the preceding claims **characterized in that** it comprises one or more fatty alcohol having 8 to 22 carbon atoms which may be saturated or unsaturated, straight, or branched.

12. Process for semi-permanent hair straightening, comprising the following steps performed in this order:
(a) application of a hair straightening composition according to claims 1 to 11 onto the hair, preferably in a weight ratio of hair to composition of 0.5:2 to 2:0.5;
(b) leaving the composition on the hair for 5 to 120 minutes at a temperature in the range of 20 to 50°C;
(c) optionally rinsing off the hair;
(d) drying the hair with a blow dryer;
(e) optionally treating the hair with an iron having a surface temperature of 180 +-50°C, preferably 170 to 200°C, and
(f) optionally rinsing off and/or shampooing the hair and drying.

13. Kit for hair comprising the composition as defined in any of the claims 1 to 11.

## Patentansprüche

1. Wässrige Zusammensetzung zur semipermanenten Glättung und/oder Volumenreduktion von Keratinfasern, insbesondere menschlichen Haaren, **dadurch gekennzeichnet, dass** sie Glyoxylsäure, deren Hydrate oder deren Salze in einer Konzentration im Bereich von 0.5 bis 30 Gew.-% enthält,
und einem Polyethylenglykolalkylether mit der allgemeinen Struktur
R2- O - (CH2CH_{(2) O})ₙH
worin R₂ eine gesättigte Alkylkette mit 9 bis 16 C-Atomen ist und n ein Wert im Bereich von 8 bis 16 ist, in einer Konzentration im Bereich von 0.5 bis 15 Gew.-%, und
es hat einen pH-Wert von 4.0 oder weniger,
wobei alle Konzentrationen auf die Gesamtmenge der Zusammensetzung berechnet werden,
wobei die Zusammensetzung frei von Reduktionsmitteln auf Schwefelbasis ist.

2. Zusammensetzung nach Anspruch 1, **dadurch gekennzeichnet, dass** der pH-Wert der Zusammensetzung 3 oder weniger beträgt, insbesondere im Bereich von 1 bis 3 liegt.

3. Zusammensetzung nach einem der vorhergehenden Ansprüche, **dadurch gekennzeichnet, dass** Polyethylenglykolalkylether eine Alkylkettenlänge von 11 bis 15 C-Atomen, vorzugsweise 12 bis 13 C-Atomen, aufweist und n einen Wert von 8 bis 14 und insbesondere 9 bis 12 hat.

4. Zusammensetzung nach einem der vorangehenden Ansprüche, **dadurch gekennzeichnet, dass** Polyethylenglykolalkylether C12-13-Pareth-9 ist.

5. Zusammensetzung nach einem der vorhergehenden Ansprüche, **dadurch gekennzeichnet, dass** sie schäumendes Tensid in einer Gesamtkonzentration von 10 Gew.-% oder weniger, berechnet auf die Gesamtmenge der Zusammensetzung, enthält.

6. Zusammensetzung nach einem der vorhergehenden Ansprüche, **dadurch gekennzeichnet, daß** sie Polyethylenglykolalkylether in einer Konzentration im Bereich von 0.5 bis 7.5 Gew.-%, berechnet auf die gesamte Zusammensetzung, enthält.

7. Zusammensetzung nach einem der vorhergehenden Ansprüche, **dadurch gekennzeichnet, dass** sie ein oder mehrere verdickende Polymere enthält, vorzugsweise ausgewählt aus anionischen Polymeren mit einer Viskosität von mindestens 500 mPa-s, gemessen bei einer Polymerkonzentration von 1 Gew.-% in Wasser und bei 20 °C mit einem Brookfield-Viskosimeter, insbesondere ausgewählt aus Xanthangummi, Hydroxypropyl-Xanthangummi und Dehydro-Xanthangummi.

8. Zusammensetzung nach einem der vorhergehenden Ansprüche, **dadurch gekennzeichnet, dass** sie ein quaternäres Ammonium-Tensid der allgemeinen Formel 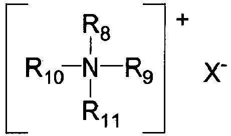
worin R₈und R₉ unabhängig voneinander eine gesättigte oder ungesättigte, verzweigte oder gerade Alkylkette mit 8-22 C-Atomen oder
R₁₂-CO-NH-(CH_{2) (n})
worin R₁₂ eine gesättigte oder ungesättigte, verzweigte oder geradkettige Alkylkette mit 7-21 C-Atomen ist und n eine ganze Zahl von 1 bis 4 ist, oder
R₁₂CO-O-(CH₂) ₍ₙ)
worin R₁₂ eine gesättigte oder ungesättigte, verzweigte oder geradkettige Alkylkette mit 7-21 C-Atomen ist und n eine ganze Zahl von 1 - 4 ist,
R₁₀ und R₁₁ sind unabhängig voneinander eine Alkylgruppe mit 1 bis 4 Kohlenstoffatomen, eine Hydroxylalkylkette mit 1 bis 4 Kohlenstoffatomen oder eine Ethoxy- oder Propoxygruppe, wobei die Anzahl der Ethoxy- oder Propoxygruppen im Bereich von 1 bis 4 variiert, und X ist Chlorid, Bromid, Methosulfat oder Ethosulfat.

9. Die Zusammensetzung nach einem der vorhergehenden Ansprüche, **dadurch gekennzeichnet, dass** sie ein oder mehrere Öle enthält, die vorzugsweise aus Silikonölen, natürlichen Ölen, Mineralölen und synthetischen Ölen ausgewählt sind.

10. Zusammensetzung nach einem der vorhergehenden Ansprüche, **dadurch gekennzeichnet, dass** sie eine Emulsion ist.

11. Zusammensetzung nach einem der vorhergehenden Ansprüche, **dadurch gekennzeichnet, dass** sie einen oder mehrere Fettalkohole mit 8 bis 22 Kohlenstoffatomen enthält, die gesättigt oder ungesättigt, gerade oder verzweigt sein können.

12. Verfahren zur semipermanenten Haarglättung, das die folgenden, in dieser Reihenfolge durchgeführten Schritte umfasst:
(a) Auftragen eines Haarglättungsmittels nach einem der Ansprüche 1 bis 11 auf das Haar, vorzugsweise in einem Gewichtsverhältnis von Haar zu Mittel von 0.5:2 bis 2:0.5;
(b) Auftragen der Zusammensetzung auf das Haar für 5 bis 120 Minuten bei einer Temperatur im Bereich von 20 bis 50°C;
(c) gegebenenfalls Ausspülen des Haares;
(d) Trocknen der Haare mit einem Fön;
(e) gegebenenfalls Behandlung des Haares mit einem Bügeleisen mit einer Oberflächentemperatur von 180 +- 50°C, vorzugsweise 170 bis 200°C, und
(f) gegebenenfalls Ausspülen und/oder Shampoonieren der Haare und Trocknen.

13. Kit für Haare, das die in einem der Ansprüche 1 bis 11 definierte Zusammensetzung enthält.

## Revendications

1. Composition aqueuse pour le lissage semi-permanent et/ou la réduction du volume des fibres kératiniques, en particulier des cheveux humains, **caractérisée par le fait qu'**elle comprend de l'acide glyoxylique, ses hydrates ou leurs sels à une concentration comprise entre 0.5 et 30 % en poids,
et un éther d'alkyle de polyéthylène glycol selon la structure générale
R₂- O - (CH₂CH_{(2) O})ₙH
dans lequel R₂est une chaîne alkyle saturée avec 9 à 16 atomes de carbone et n est une valeur comprise entre 8 et 16 à une concentration comprise entre 0.5 et 15 % en poids, et
il a un pH inférieur ou égal à 4.0,
toutes les concentrations étant calculées par rapport au total de la composition, dans laquelle la composition est exempte d'agents réducteurs à base de soufre.

2. Composition selon la revendication 1 **caractérisée par le fait que** le pH de la composition est inférieur ou égal à 3, notamment compris entre 1 et 3.

3. Composition selon l'une quelconque des revendications précédentes **caractérisée par le fait que** l'éther d'alkyle du polyéthylène glycol a une longueur de chaîne alkyle de 11 à 15 atomes de carbone, de préférence de 12 à 13 atomes de carbone et que n est une valeur de 8 à 14 et en particulier de 9 à 12.

4. Composition selon l'une quelconque des revendications précédentes, **caractérisée par le fait que** l'éther d'alkyle du polyéthylène glycol est C12-13 pareth-9.

5. La composition selon l'une des revendications précédentes est **caractérisée par le fait qu'**elle comprend un agent tensioactif moussant à une concentration totale inférieure ou égale à 10 % en poids, calculée par rapport au total de la composition.

6. La composition selon l'une des revendications précédentes est **caractérisée par le fait qu'**elle comprend l'éther alkyl de polyéthylène glycol à une concentration comprise entre 0.5 et 7.5 % en poids, calculée par rapport au total de la composition.

7. La composition selon l'une quelconque des revendications précédentes **caractérisée en ce qu'**elle comprend un ou plusieurs polymères épaississants, de préférence choisis parmi les polymères anioniques ayant une viscosité d'au moins 500 mPa-s mesurée à une concentration de polymère de 1% en poids, dans l'eau et à 20°C avec un viscosimètre Brookfield, en particulier choisis parmi la gomme xanthane, la gomme hydroxypropyl xanthane et la gomme déhydro xanthane.

8. Composition selon l'une quelconque des revendications précédentes, **caractérisée par le fait qu'**elle comprend un tensioactif à base d'ammonium quaternaire de formule générale 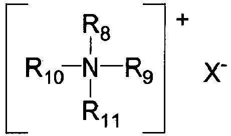
dans lequel R₈ et R₉ sont, indépendamment l'un de l'autre, une chaîne alkyle saturée ou insaturée, ramifiée ou linéaire, comportant 8 à 22 atomes de carbone, ou
R₁₂-CO-NH-(CH_{2) (n})
dans lequel R₁₂ est une chaîne alkyle saturée ou insaturée, ramifiée ou linéaire, comportant 7 à 21 atomes de carbone, et n est un nombre entier compris entre 1 et 4, ou
R₁₂CO-O-(CH_{2) (n})
où R₁₂ est une chaîne alkyle saturée ou insaturée, ramifiée ou linéaire, comportant 7 à 21 atomes de carbone, et n est un nombre entier compris entre 1 et 4,
R₁₀et R₁₁sont indépendamment l'un de l'autre un groupe alkyle avec 1 à 4 atomes de carbone, une chaîne alkyle hydroxyle avec 1 à 4 atomes de carbone, ou un groupe éthoxy ou propoxy avec un nombre de groupes éthoxy ou propoxy variant dans la gamme de 1 à 4, et X est le chlorure, le bromure, le méthosulfate ou l'éthosulfate.

9. La composition selon l'une des revendications précédentes est **caractérisée par le fait qu'**elle comprend une ou plusieurs huiles, de préférence choisies parmi les huiles de silicone, les huiles naturelles, les huiles minérales et les huiles synthétiques.

10. Composition selon l'une des revendications précédentes, **caractérisée par le fait qu'**il s'agit d'une émulsion.

11. La composition selon l'une des revendications précédentes est **caractérisée par le fait qu'**elle comprend un ou plusieurs alcools gras ayant de 8 à 22 atomes de carbone, qui peuvent être saturés ou insaturés, droits ou ramifiés.

12. Procédé de lissage semi-permanent des cheveux, comprenant les étapes suivantes effectuées dans cet ordre :
(a) application d'une composition lissante selon les revendications 1 à 11 sur les cheveux, de préférence dans un rapport en poids entre les cheveux et la composition de 0.5:2 à 2:0.5 ;
(b) laisser la composition sur les cheveux pendant 5 à 120 minutes à une température comprise entre 20 et 50°C ;
(c) rincer éventuellement les cheveux ;
(d) le séchage des cheveux à l'aide d'un sèche-cheveux ;
(e) éventuellement, traiter les cheveux avec un fer à repasser dont la température de surface est de 180 +- 50°C, de préférence de 170 à 200°C, et
(f) éventuellement, rincer et/ou shampouiner les cheveux et les sécher.

13. Kit pour cheveux comprenant la composition telle que définie dans l'une des revendications 1 à 11.
